# EUROPEAN PATENT APPLICATION

(11) **EP 4 205 687 A1**
(43) Date of publication of application: **05.07.2023**
(21) Application number: 20953279.5
(22) Date of filing: 10.09.2020
(51) Int. Cl.: A61B 34/30, A61B 17/28

(54) **FORCEPS DEVICE**

(71) Applicant: RIVERFIELD Inc., 1070052 Tokyo (JP)
(72) Inventor: TAKIKAWA, Kyohei, Tokyo 160-0017 (JP)
(74) Representative: adares Patent- und Rechtsanwälte Reininger & Partner GmbB
(86) International application number: PCT/JP2020/034370
(87) International publication number: WO 2022/054214

(57) **Abstract**

A forceps device (10) includes a grasping part (12a, 12b), a support (14) that holds the grasping part (12a, 12b), a first rotating shaft (16) that turnably supports the support (14), a base member that holds the first rotating shaft (16), a plurality of guide pulleys (20) arranged coaxially with the first rotating shaft (16), a plurality of wires (26, 28) running over the guide pulleys (20) and the grasping part without other pulleys therebetween and transmitting driving force to move the grasping part, a support pulley (42) provided on the support (14), and a support wire (38, 40) running over the support pulley (42) and transmitting driving force to turn the support about the first rotating shaft (16).

## Description

### Technical Field

The present invention relates to a forceps device used for a manipulator of a surgical robot.

### Background Art

Medical treatments using robots (manipulators) have recently been proposed in order to reduce the burden on operators and save manpower in medical facilities. In the field of surgery, proposals have been made for surgical manipulator systems for operators to treat patients by operating remotely-controllable surgical manipulators.

Various surgical tools are attached to leading ends of surgical manipulators. Known surgical tools include forceps for grasping human tissue during surgery. For example, Patent Literature 1 teaches forceps including two jaw parts serving as grasping portions, two disks having different outer diameters from each other provided on the respective jaw parts, and wires running over the disks. The wires are pulled to open or close the jaw parts.

### Related Art List

### Patent Literature

Patent Literature 1: US 2015/0127045 A1

### Summary of Invention

### Technical Problem

In the aforementioned forceps, however, deflection rollers for making fleet angles of the wires smaller are provided between a disk over which a wire that is pulled for bending the whole jaw parts and a disk provided on the jaw parts. Thus, some distance is needed between a rotation axis of bending of the whole jaw parts and a rotation axis of opening and closing of the jaw parts. The torque therefore reduces at the leading end of the forceps and the controllability thus lowers as compared with forceps with a short distance between the rotation axes.

The present invention has been made in view of the aforementioned circumstances, and an exemplary object thereof is to provide a forceps device with high controllability.

### Solution to problem

To solve the aforementioned problems, a forceps device according to an aspect of the present invention includes: a grasping part; a support that holds the grasping part; a first rotating shaft that turnably supports the support; a base member that holds the first rotating shaft; a plurality of guide pulleys arranged coaxially with the first rotating shaft; a plurality of grasping portion wires running over the guide pulleys and the grasping part without other pulleys therebetween and transmitting driving force to move the grasping part; a support pulley provided on the support; and a support wire running over the support pulley and transmitting driving force to turn the support about the first rotating shaft.

According to this aspect, the distance between the first rotating shaft and the second rotating shaft can be shortened, and the torque of the grasping part can therefore be increased. As a result, a forceps device with high controllability of the grasping part can be provided.

The grasping part may include a first grasping portion and second grasping portion that move relative to each other to grasp an object. The first grasping portion may be continuous with a first grasping portion pulley and a second grasping portion pulley, which constitute a bifurcated shape, and the second grasping portion may be continuous with a third grasping portion pulley and a fourth grasping portion pulley, which constitute a bifurcated shape. The forceps device may further include a second rotating shaft that rotatably supports the first grasping portion pulley, the third grasping portion pulley, the second grasping portion pulley, and the fourth grasping portion pulley arranged in this order.

Another aspect of the present invention is also directed to a forceps device. The device includes: a grasping part; a support that holds the grasping part; a first rotating shaft that turnably supports the support; a base member that holds the first rotating shaft; a plurality of guide pulleys arranged coaxially with the first rotating shaft; and a plurality of grasping portion wires running over the guide pulleys and the grasping part without other pulleys therebetween and transmitting driving force to move the grasping part. The grasping part includes a first grasping portion and second grasping portion that move relative to each other to grasp an object. The first grasping portion is continuous with a first grasping portion pulley and a second grasping portion pulley, which constitute a bifurcated shape, and the second grasping portion is continuous with a third grasping portion pulley and a fourth grasping portion pulley, which constitute a bifurcated shape. The forceps device further includes a second rotating shaft that rotatably supports the first grasping portion pulley, the third grasping portion pulley, the second grasping portion pulley, and the fourth grasping portion pulley arranged in this order.

According to this aspect, the distance between the first rotating shaft and the second rotating shaft can be shortened, and the torque of the grasping part can therefore be increased. As a result, a forceps device with high controllability of the grasping part can be provided.

The support may include a cylindrical part through which the first rotating shaft extends. The guide pulleys are rotatably supported by an outer circumference of the cylindrical part.

The guide pulleys may include first to fourth guide pulleys. The grasping portion wires may be passed over the first to fourth guide pulleys and the first to fourth grasping portion pulleys without other pulleys therebetween.

The grasping portion wires may include: a first grasping portion wire running over the first guide pulley and the third grasping portion pulley; a second grasping portion wire running over the second guide pulley and the first grasping portion pulley; a third grasping portion wire running over the third guide pulley and the second grasping portion pulley; and a fourth grasping portion wire running over the fourth guide pulley and the fourth grasping portion pulley.

The first grasping portion wire may be passed over a first side of the first guide pulley with respect to a vertical cross section including the first rotating shaft and being perpendicular to the second rotating shaft, and then fixed to the third grasping portion pulley located on the first side with respect to the vertical cross section, the second grasping portion wire may be passed over the first side of the second guide pulley with respect to the vertical cross section, and then fixed to the first grasping portion pulley located on the first side with respect to the vertical cross section, the third grasping portion wire may be passed over a second side of the third guide pulley with respect to the vertical cross section, and then fixed to the second grasping portion pulley located on the second side with respect to the vertical cross section, and the fourth grasping portion wire may be passed over the second side of the fourth guide pulley with respect to the vertical cross section, and then fixed to the fourth grasping portion pulley with respect to the vertical cross section. As a result, the four grasping portion wires are fixed to the four grasping portion pulleys, respectively, without intersecting with each other. In addition, the four grasping portion wires running over the first to fourth guide pulleys are fixed to the four grasping portion pulleys, respectively, with small fleet angles.

The aforementioned forceps device may further include: a fifth guide pulley located on an upstream side of the first guide pulley and on the second side with respect to the vertical cross section; a sixth guide pulley located on an upstream side of the second guide pulley and on the second side with respect to the vertical cross section; a seventh guide pulley located on an upstream side of the third guide pulley and on the first side with respect to the vertical cross section; and an eighth guide pulley located on an upstream side of the fourth guide pulley and on the first side with respect to the vertical cross section. As a result, when the support is bent in either direction about the first rotating shaft, one or more of the grasping portion wires come in contact with the associated one or more of the first to fourth guide pulleys, which stabilizes the controllability when the support is turned.

Note that any combination of the components described above, and any expression in the present invention converted to that for a method, a device, a system, and the like also remain as aspects of the present invention.

### Advantageous Effects of Invention

According to the present invention, a forceps device with high controllability can be achieved.

### Brief Description of Drawings

FIG. 1 is a perspective view of a forceps device according to an embodiment.
FIG. 2 is a front view of the forceps device of FIG. 1 as viewed in a direction A.
FIG. 3 is a side view of the forceps device of FIG. 1 as viewed in a direction B.
FIG. 4 is a side view of the forceps device of FIG. 1 as viewed in a direction C.
FIG. 5 is a cross-sectional view of the forceps device illustrated in FIG. 3 along D-D.
FIG. 6 is an enlarged view of a region R in FIG. 5.
FIG. 7 is a front view of a guide pulley according to the embodiment.
FIG. 8 is a cross-sectional view of the guide pulley illustrated in FIG. 7 along E-E.
FIG. 9 is a perspective view of a support according to the embodiment.
FIG. 10 is a flowchart explaining a method for manufacturing the forceps device according to the embodiment.
FIG. 11 is a perspective view illustrating a state in which a pair of jaw parts facing each other are to be fitted according to the embodiment.
FIG. 12 is a perspective view of the forceps device illustrated in FIG. 1 as viewed in a direction H1.
FIG. 13 is a perspective view of the forceps device illustrated in FIG. 1 as viewed in a direction H2.
FIG. 14 is a perspective view of the forceps device illustrated in FIG. 1 as viewed in a direction H3.
FIG. 15 is a perspective view of the forceps device illustrated in FIG. 1 as viewed in a direction opposite the direction H2.
FIG. 16 is a view of the forceps device illustrated in FIG. 3 without illustration of a base member.
FIG. 17 is a view of the forceps device illustrated in FIG. 4 without illustration of the base member.

### Description of Embodiments

The present invention will now be described on the basis of an embodiment with reference to the drawings. Components, members, and processes that are the same as or equivalent to each other illustrated in the drawings are represented by the same reference numerals, and redundant explanation will not be repeated where appropriate. The embodiment is not to limit the invention, but is an example, and any feature or any combination of features described in the embodiment is not necessarily essential to the invention.

### [Forceps device]

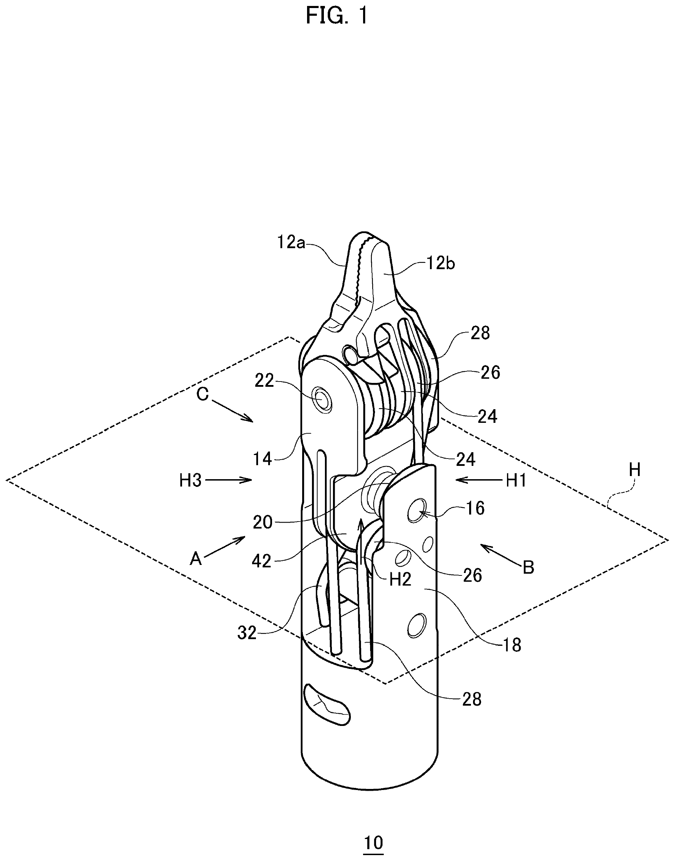

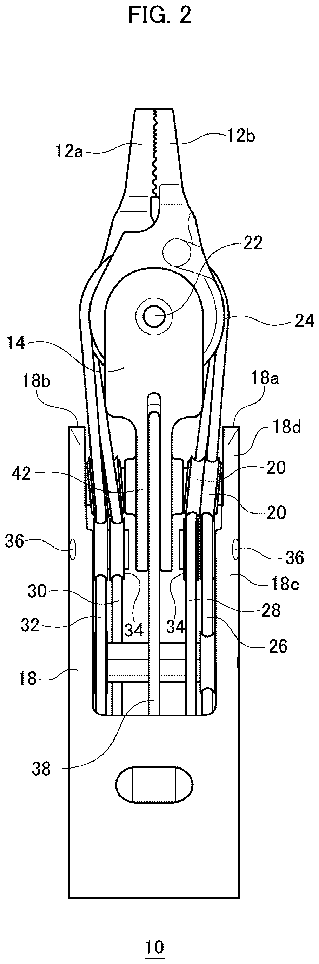

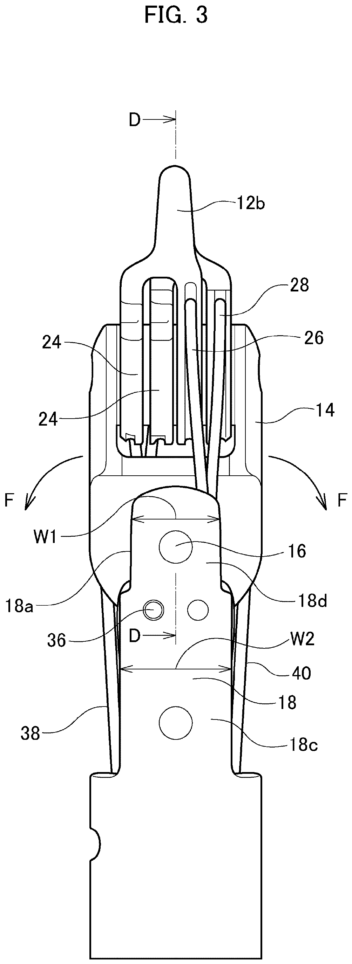

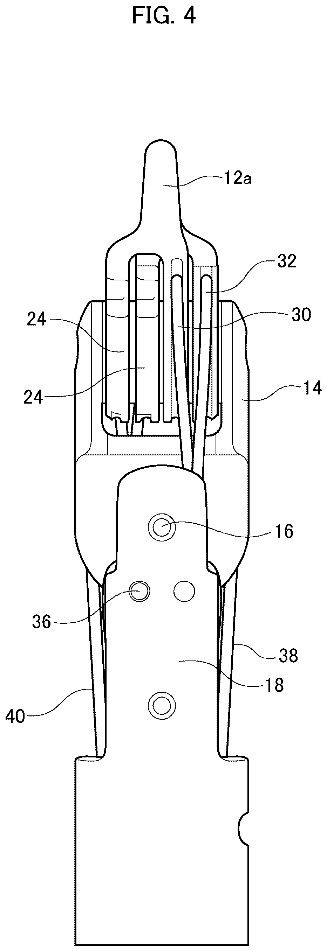

FIG. 1 is a perspective view of a forceps device according to an embodiment. FIG. 2 is a front view of the forceps device of FIG. 1 as viewed in a direction A. FIG. 3 is a side view of the forceps device of FIG. 1 as viewed in a direction B. FIG. 4 is a side view of the forceps device of FIG. 1 as viewed in a direction C.

The forceps device 10 illustrated in the drawings includes a pair of grasping portions 12a and 12b, a support 14 that holds the pair of grasping portions 12a and 12b, a first rotating shaft 16 that turnably supports the support 14, a base member 18 that holds the first rotating shaft 16, four guide pulleys 20 arranged coaxially with the first rotating shaft 16, a second rotating shaft 22 that turnably supports the pair of grasping portions 12a and 12b and is held by the support 14, four jaw pulleys 24 supported coaxially with the second rotating shaft 22, four wires 26, 28, 30 and 32 running over the four guide pulleys 20 and the four jaw pulleys 24, and wires 38 and 40 for rotating the support 14 about the first rotating shaft 16.

### [Guide pulleys 20]

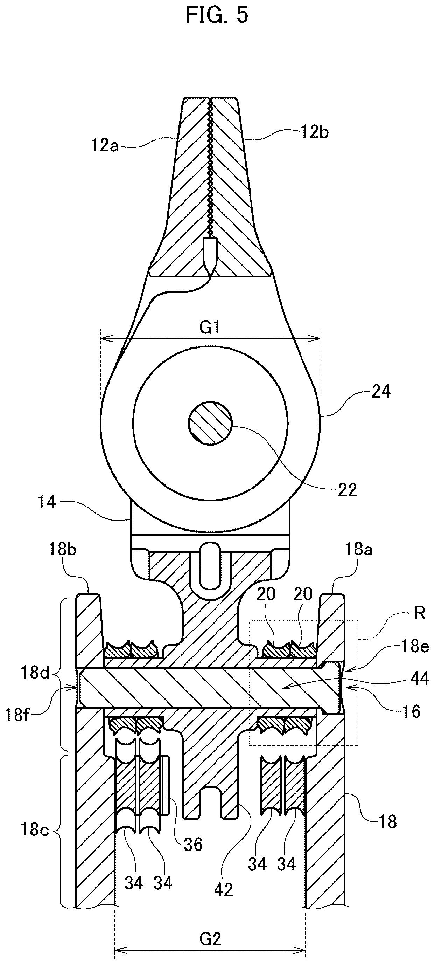

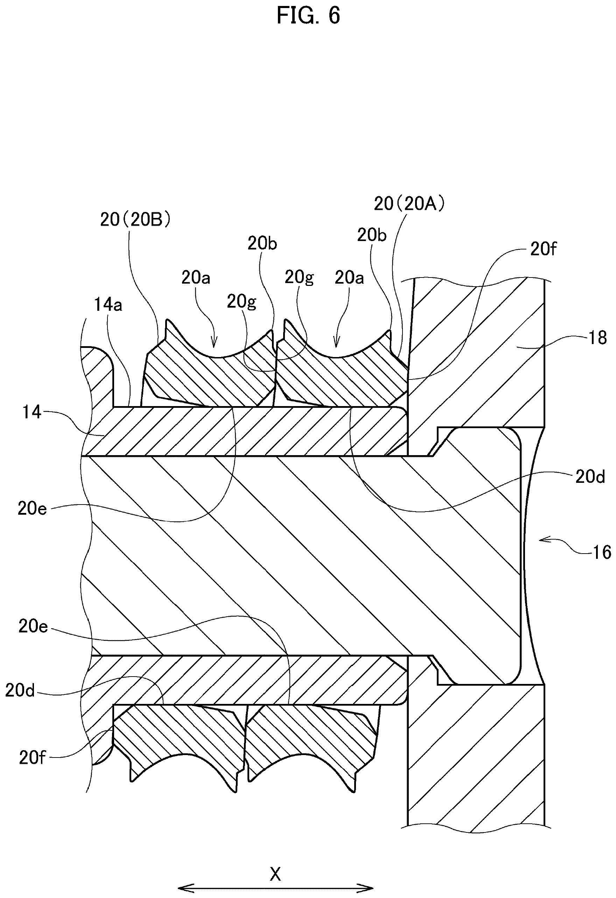

FIG. 5 is a cross-sectional view of the forceps device 10 illustrated in FIG. 3 along D-D. FIG. 6 is an enlarged view of a region R in FIG. 5. As illustrated in FIGS. 5 and 6, the guide pulleys 20 are rotatably supported by the first rotating shaft 16 with an annular part 14a having a cylindrical shape, which is part of the support 14, therebetween and at an angle with respect to the first rotating shaft 16. Thus, the respective wires 26, 28, 30 and 32 have small substantial fleet angles with respect to the guide pulleys 20, and therefore become less likely to interfere with edges 20b of guide grooves 20a of the guide pulleys 20. Alternatively, the guide pulleys 20 may be directly supported by the first rotating shaft 16 without the support 14 therebetween.

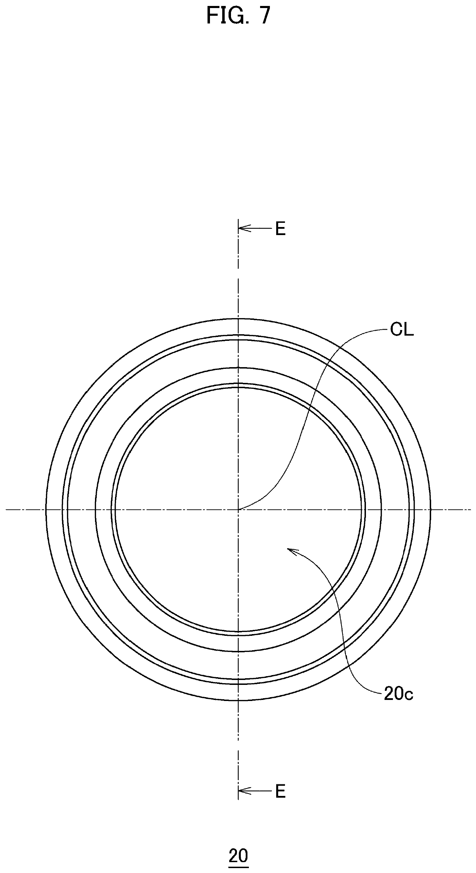

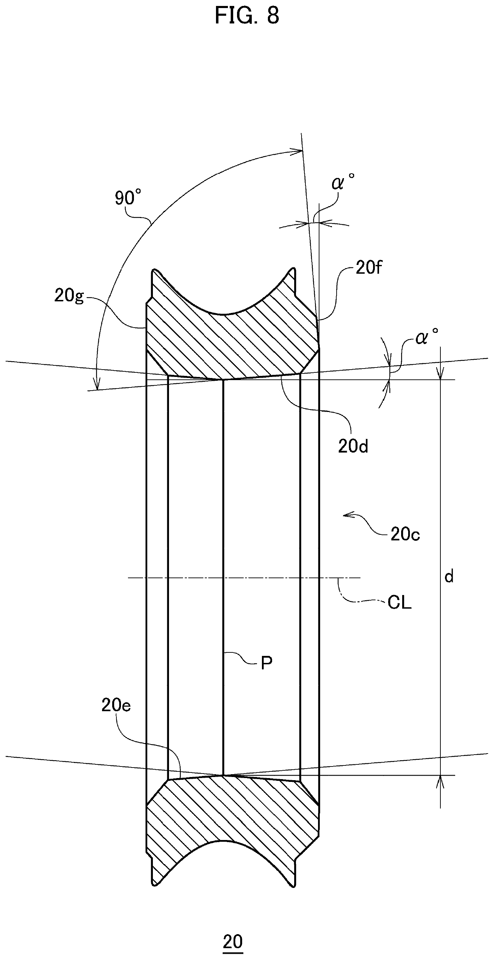

FIG. 7 is a front view of a guide pulley 20 according to the embodiment. FIG. 8 is a cross-sectional view of the guide pulley 20 illustrated in FIG. 7 along E-E. Each guide pulley 20 according to the embodiment has an inner circumferential face 20d tilted with respect to a center line CL of a circular opening 20c thereof through which the first rotating shaft 16 extends. The inner circumferential face 20d slides relative to the annular part 14a of the support 14 into which the first rotating shaft 16 is inserted. Thus, the guide pulleys 20 are autonomously tilted due to the forces received from wires having fleet angles. In a case where the guide pulleys 20 are supported directly by the first rotating shaft 16, the inner circumferential face 20d of each guide pulley 20 slides directly relative to the outer circumferential face of the first rotating shaft 16.

As illustrated in FIG. 8, the inner circumferential face 20d of each guide pulley 20 is formed so that the inner diameter d of the guide pulley 20 gradually increases from the position of a plane P, which is perpendicular to the center line CL and at which the inner diameter d is smallest, as the position is farther along the center line CL from the plane P. In other words, the inner circumferential face 20d may have a conical shape, an inverted cone shape, or a tapered shape. In addition, an inner circumferential face 20e opposite the inner circumferential face 20d with respect to the plane P is also processed to have a shape similar to the inner circumferential face 20d.

Note that each of the guide pulleys 20 according to the embodiment is formed such that the angle α between the inner circumferential face 20d or the inner circumferential face 20e and the center line CL is within a range from 3 to 7 degrees. In addition, one end face 20f of each guide pulley 20 is tilted at the angle α with respect to the plane P, and the angle between the end face 20f and the inner circumferential face 20d is 90°. Furthermore, the other end face 20g of the guide pulley 20 is parallel to the plane P (perpendicular to the center line CL). Each guide pulley 20 is therefore an annular member that is asymmetric with respect to the plane P.

As illustrated in FIGS. 5 and 6, the guide pulleys 20 according to the embodiment are arranged in such a manner that two guide pulleys 20 are arranged adjacent to each other on each side of the support 14 with the support 14 therebetween. In addition, as illustrated in FIG. 6, the end faces 20g of two guide pulleys 20 that are arranged adjacent to each other on the annular part 14a face each other. As a result, one (a guide pulley 20B) of two guide pulleys 20 that are adjacent to each other is in contact with and tilted with respect to the support 14, and the other (a guide pulley 20A) of the two guide pulleys 20 adjacent to each other is in contact with and tilted with respect to the base member 18. Thus, when a guide pulley 20 is tilted, the guide pulley 20 is accurately positioned in a first rotating shaft direction X.

As illustrated in FIG. 2, the guide pulleys 20 are each tilted with respect to the first rotating shaft 16 so that the wires 26 and 28 running over the guide pulleys 20 extend out toward the outer circumference of the jaw pulleys 24. As a result, even in a case where jaw pulleys 24 having a large diameter that are likely to increase the operation torque of the pair of grasping portions 12a and 12b are used, interference of wires at the guide pulleys 20 can be reduced.

Next, the base member 18 will be described. As illustrated in FIG. 2 and FIG. 5, the base member 18 according to the embodiment includes a pair of arms 18a and 18b that hold respective ends of the first rotating shaft 16, and third rotating shafts 36 that are held by the pair of arms 18a and 18b, respectively, and rotatably support four guide pulleys 34 located upstream of the guide pulleys 20. Note that a third rotating shaft 36 according to the embodiment is provided on each of the pair of arms 18a and 18b.

The arms 18a and 18b each have a base part 18c holding the third rotating shaft 36, and a distal end part 18d that holds the first rotating shaft 16 and that is thinner than the base part 18c. In other words, the distance between the distal end parts 18d is larger than the distance between the base parts 18c. Thus, as illustrated in FIG. 3, even when the wires 26 and 28 having the fleet angles are bent together with the support 14 around the first rotating shaft 16 (in the direction of an arrow F in FIG. 3), the wires 26 and 28 are less likely to interfere with the arms 18a and 18b.

Furthermore, the circumferential width W1 of the distal end part 18d of the arm 18a (the arm 18b) is smaller than the circumferential width W2 of the base part 18c thereof. Thus, a U-shaped recess is formed at the distal end part 18d at which interference with a wire needs to be addressed, and the circumferential width of the distal end part 18d is made larger than the circumferential width of the base part 18c, which minimizes deterioration of the stiffness of the arm.

In addition, as illustrated in FIG. 5, the jaw pulleys 24 according to the embodiment have an outer diameter G1, which is larger than the distance between the base parts 18c of the pair of arms 18a and 18b. Thus, in the forceps device 10 according to the embodiment, the jaw pulleys 24 having a large outer diameter relative to the inner diameter of the cylindrical base member 18 may be used.

### [Easiness of assembly of support]

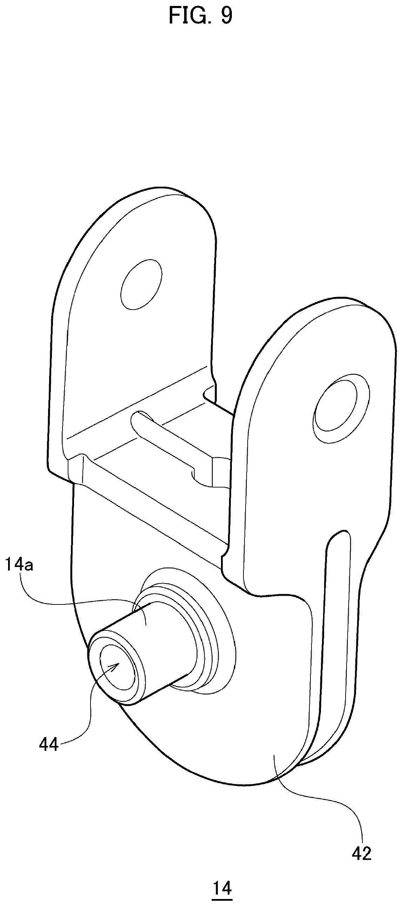

FIG. 9 is a perspective view of the support according to the embodiment. As illustrated in FIGS. 5 and 6, the forceps device 10 according to the embodiment includes the pair of grasping portions 12a and 12b, the support 14 that holds the pair of grasping portions 12a and 12b, the first rotating shaft 16 that turnably supports the support 14, the base member 18 that holds the first rotating shaft 16, and a plurality of guide pulleys 20 arranged coaxially with the first rotating shaft 16. As illustrated in FIG. 9, the support 14 has the annular part 14a, which is a cylindrical part through which the first rotating shaft 16 extends. The guide pulleys 20 are rotatably supported by the outer circumference of the annular part 14a. Note that each rotating shaft is not limited to a shaft that rotates by itself, but may be any shaft that is the center of rotation of a member supported thereby, and may be a shaft fixed to another member.

In the forceps device 10 having such a structure, the support 14 in a state in which a plurality of guide pulleys 20 are supported by the outer circumference of the annular part 14a is held by the base member 18 with the first rotating shaft 16 therebetween. This configuration facilitates improvement in the easiness of assembly as compared with a case where the support 14 is held directly by the base member 18.

In addition, the base member 18 of the embodiment has the pair of arms 18a and 18b facing each other. The first rotating shaft 16 is firmly fixed in such a manner that the axial ends thereof are press-fitted to the pair of arms 18a and 18b. As a result, because the distal ends of the pair of arms 18a and 18b, which can be free ends, are fixed by the rotating shaft, the stiffness of the whole base member 18 increases.

Each of the wires 26, 28, 30 and 32 transmits a driving force to the grasping portion 12a or the grasping portion 12b to move the grasping portion 12a or the grasping portion 12b. Specifically, the wire 26 and the wire 32 run over the jaw pulleys 24 for the grasping portion 12b, and the grasping portion 12b moves in an opening direction when the wire 26 is pulled and moves in a closing direction when the wire 32 is pulled. The wire 28 and the wire 30 run over the jaw pulleys 24 for the grasping portion 12a, and the grasping portion 12a moves in a closing direction when the wire 28 is pulled and moves in an opening direction when the wire 30 is pulled.

In addition, each of the four guide pulleys 20 has a corresponding one of the wires 26, 28, 30 and 32 placed thereover. Each of the wires 26, 28, 30 and 32 runs over the corresponding one of the guide pulleys 20 so that the normal force applied to the support 14 from the first rotating shaft 16 is reduced when the grasping portions 12a and 12b are moved. More specifically, as illustrated in FIG. 2, each of the wires 26, 28, 30 and 32 passes between the corresponding ones of the guide pulleys 20 and guide pulleys 34 and runs over the corresponding one of the guide pulleys 20 from a lower side thereof (a side opposite the grasping portion 12a or 12b with respect to the first rotating shaft 16) .

Thus, when tension is applied to at least any one of the wires 26, 28, 30 and 32 for moving the grasping portions 12a and 12b, the tension causes a force acting on the support 14 via the guide pulleys 20. In the case of the forceps device 10 according to the embodiment, the tension applied to the wires 26, 28, 30 and 32 causes a force acting on the support 14 in directions toward the grasping portions 12a and 12b.

The support 14 is formed integrally with a support pulley 42 over which the wires 38 and 40 for transmitting a driving force for rotating the support about the first rotating shaft 16 run. Thus, when one of the wires 38 and 40 is pulled to turn the support 14, the tension of the wire 38 or 40 running over the support pulley 42 presses the support 14 toward the first rotating shaft 16. A normal force received by the support 14 from the first rotating shaft 16 is thus generated, which contributes to an increase in frictional force.

In the forceps device 10 according to the embodiment, however, the tension of the wires 26, 28, 30 and 32 for moving the grasping portions 12a and 12b causes a force acting on the guide pulleys 20 in the upward direction in FIG. 2 as described above. The normal force caused by the wires 38 and 40 is reduced by the force acting on the guide pulleys 20, which reduces the frictional force when the support 14 turns about the first rotating shaft 16. Consequently, the movement of the grasping portions 12a and 12b becomes smoother, and the controllability of the forceps device 10 improves.

### [Method for producing forceps device]

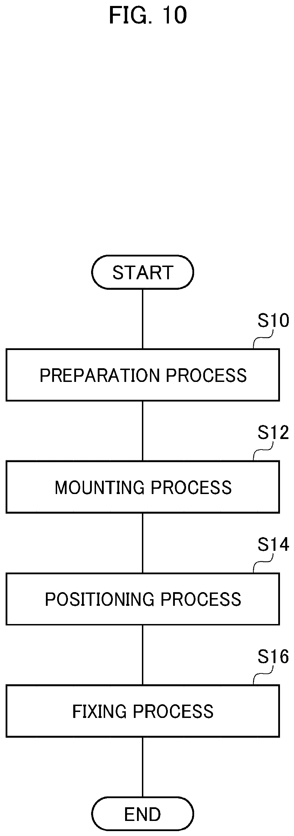

Next, a method for producing the forceps device 10 according to the embodiment will be explained. FIG. 10 is a flowchart explaining a method for manufacturing the forceps device according to the embodiment. In the method, the support 14 holding the grasping portions 12a and 12b is first prepared (S10). Subsequently, a plurality of guide pulleys 20 are mounted on the outer circumference of the annular part 14a through which a through-hole 44 (see FIG. 5) is formed (S12). The first rotating shaft 16 that turnably supports the support 14 is to be inserted in the through-hole 44. Subsequently, the support 14 is positioned so that holes 18e and 18f into which the first rotating shaft 16 is to be inserted and the through-hole 44 are linearly aligned with respect the base member 18 through which the holes 18e and 18f are formed (S14). The first rotating shaft 16 is then inserted into the hole 18e, and the respective ends of the first rotating shaft 16 are ultimately press-fitted into the holes 18e and 18f of the base member 18, so that the first rotating shaft 16 is firmly fixed to the base member 18 (S16). Note that a process of passing wires over the pulleys may be performed at a timing that does not interfere with the manufacture described above.

According to the manufacturing method, such a process as sandwiching a support by base member parts into which a base member is divided and then bonding the base member parts to each other need not be performed. In addition, such a process as coaxially arranging holes of a support, pulleys and a base member, and press-fitting a rotating shaft into the support while keeping the positions of the holes, which involves difficult adjustment, need not be performed.

### [Grasping portions]

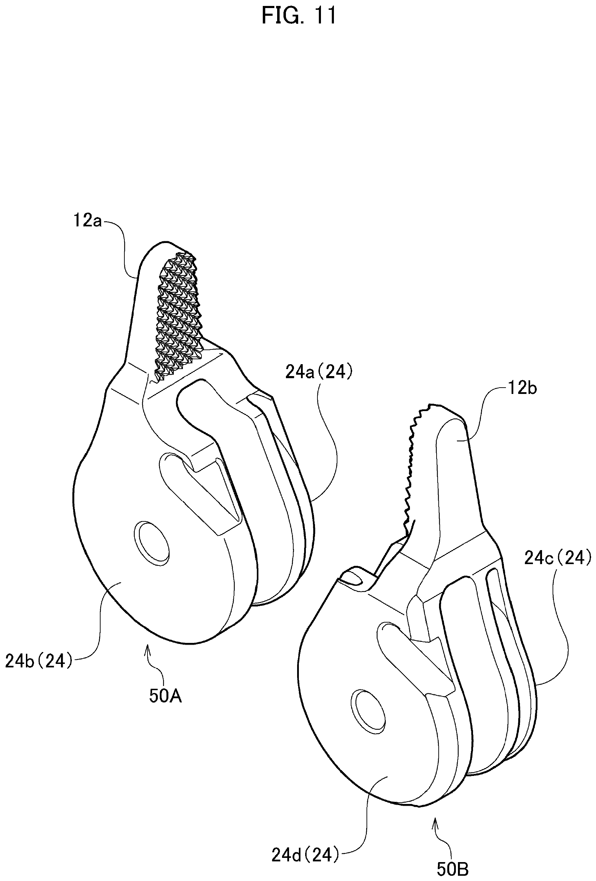

Next, jaw parts constituting the grasping portions according to the embodiment will be described in detail. FIG. 11 is a perspective view illustrating a state in which a pair of jaw parts facing each other are to be fitted according to the embodiment. Jaw parts 50A and 50B illustrated in FIG. 11 are parts having substantially the same shapes as each other. The jaw parts 50A and 50B have the grasping portion 12a and the grasping portion 12b, respectively, which move relative to each other to grasp an object. The grasping portion 12a is continuous with a jaw pulley 24a (first grasping portion pulley) and a jaw pulley 24b (second grasping portion pulley), which are formed in a bifurcated shape, and the grasping portion 12a and the jaw pulleys 24a and 24b integrally constitute the jaw part 50A. In addition, the grasping portion 12b is continuous with a jaw pulley 24c (third grasping portion pulley) and a jaw pulley 24d (fourth grasping portion pulley), which are formed in a bifurcated shape, and the grasping portion 12b and the jaw pulleys 24c and 24d integrally constitute the jaw part 50B.

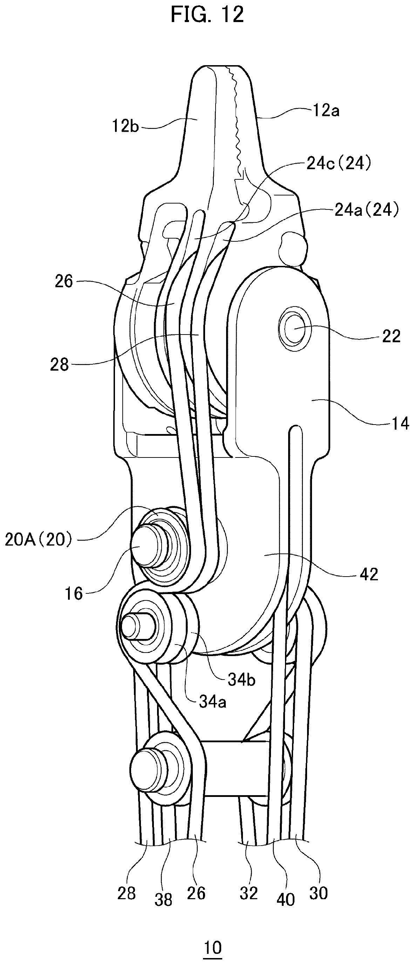

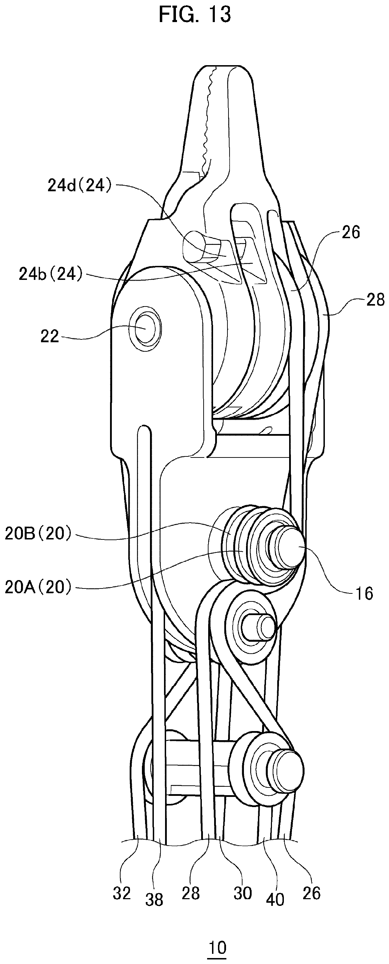

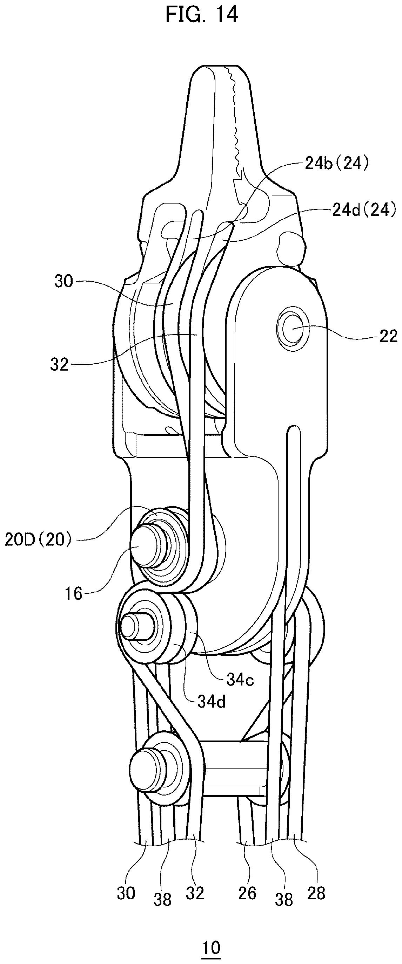

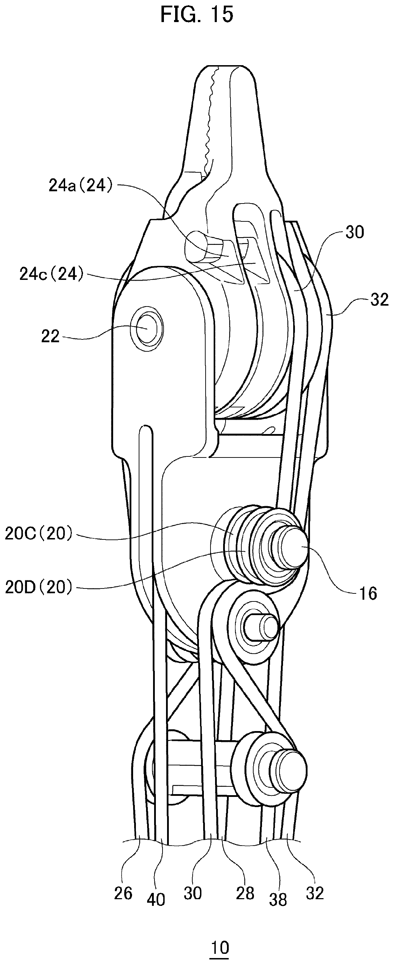

FIG. 12 is a perspective view of the forceps device 10 illustrated in FIG. 1 as viewed in a direction H1. FIG. 13 is a perspective view of the forceps device 10 illustrated in FIG. 1 as viewed in a direction H2. FIG. 14 is a perspective view of the forceps device 10 illustrated in FIG. 1 as viewed in a direction H3. FIG. 15 is a perspective view of the forceps device 10 illustrated in FIG. 1 as viewed in a direction opposite the direction H2. Note that the directions H1 to H3 are directions within a horizontal plane H including the first rotating shaft 16. In addition, the base member 18 is not illustrated in FIGS. 12 to 15.

As illustrated in FIGS. 12 to 15, the jaw pulley 24a, the jaw pulley 24c, the jaw pulley 24b, and the jaw pulley 24d are rotatably supported in this order by the second rotating shaft 22. In addition, the guide pulley 20A, the guide pulley 20B, the guide pulley 20C, and the guide pulley 20D are rotatably supported in this order by the first rotating shaft 16. The wires 26, 28, 30 and 32, which are grasping portion wires for transmitting driving forces for causing the grasping portions 12a and 12b to perform opening and closing movements, run between the guide pulleys 20A to 20D and the grasping portions 12a and 12b without other pulleys. Note that the driving forces are input from an actuator unit outside of the forceps device 10. This configuration can shorten the distance between the first rotating shaft 16 and the second rotating shaft 22 as illustrated in FIG. 12, etc., and can therefore increase the torque (operation force) of the grasping portions 12a and 12b. Consequently, the forceps device 10 with high controllability of the grasping portions 12a and 12b can be provided.

### [How wires run over pulleys]

Next, the manner in which the wires 26, 28, 30 and 32 according to the embodiment run over the pulleys will be described in detail. The wire 26 runs between the guide pulley 20A and the jaw pulley 24c. The wire 28 runs between the guide pulley 20B and the jaw pulley 24a. The wire 30 runs between the guide pulley 20C and the jaw pulley 24b. The wire 32 runs between the guide pulley 20D and the jaw pulley 24d.

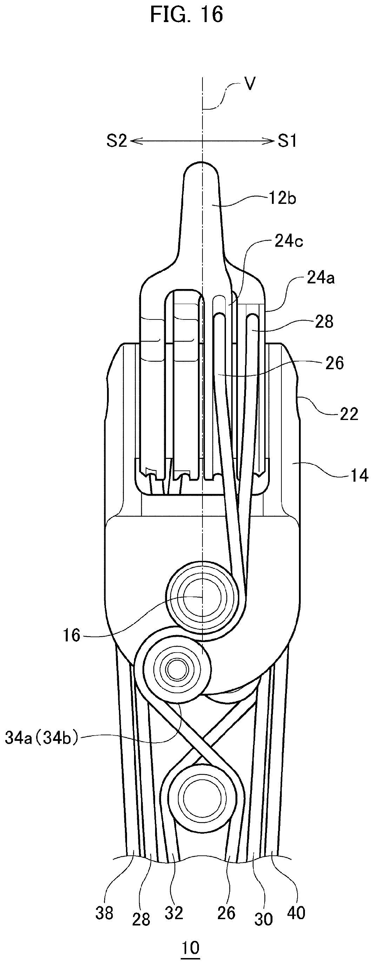

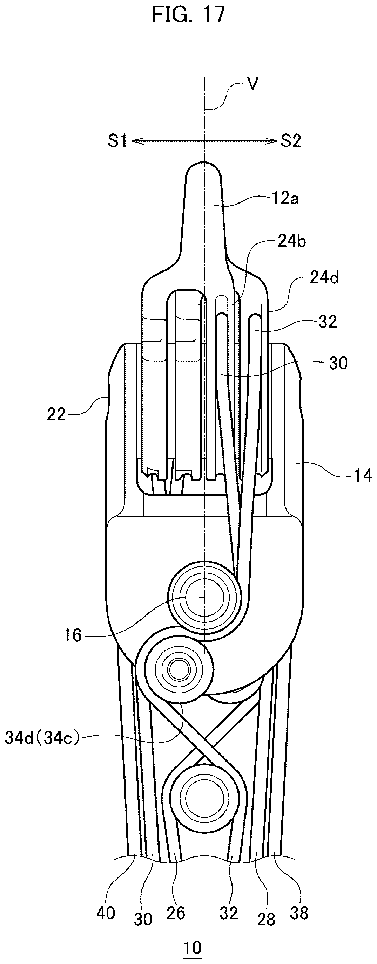

FIG. 16 is a view of the forceps device 10 illustrated in FIG. 3 without illustration of the base member 18. FIG. 17 is a view of the forceps device 10 illustrated in FIG. 4 without illustration of the base member.

As illustrated in FIGS. 12, 13, and 16, the wire 26 is passed over a first side S1 of the guide pulley 20A with respect to a vertical cross section V including the first rotating shaft 16 and being perpendicular to the second rotating shaft 22, and then fixed to the jaw pulley 24c located on the first side S1 with respect to the vertical cross section V. The wire 28 is passed over the first side S1 of the guide pulley 20B with respect to the vertical cross section V, and then fixed to the jaw pulley 24a located on the first side S1 with respect to the vertical cross section V.

In addition, as illustrated in FIGS. 14 to 16, the wire 30 is passed over the second side S2 of the guide pulley 20C with respect to the vertical cross section V, and then fixed to the jaw pulley 24b located on the second side with respect to the vertical cross section V. The wire 32 is passed over the second side S2 of the guide pulley 20D with respect to the vertical cross section V, and then fixed to the jaw pulley 24d located on the second side S2 with respect to the vertical cross section V. As a result, the four grasping portion wires are fixed to the four grasping portion pulleys, respectively, without intersecting with each other. In addition, the four grasping portion wires running over the guide pulleys 20A to 20D are fixed to the four grasping portion pulleys, respectively, with small fleet angles.

Furthermore, the forceps device 10 includes the guide pulley 34a on the upstream side of the guide pulley 20A (on the side opposite the grasping portions) and on the second side S2 with respect to the vertical cross section V, the guide pulley 34b on the upstream side of the guide pulley 20B and on the second side S2 with respect to the vertical cross section V, the guide pulley 34c on the upstream side of the guide pulley 20C and on the first side S1 with respect to the vertical cross section V, and the guide pulley 34d on the upstream side of the guide pulley 20D and on the first side S1 with respect to the vertical cross section V.

As a result, when the support 14 is bent in either direction about the first rotating shaft 16, one or more of the wires 26, 28, 30 and 32 come in contact with the associated one or more of the guide pulleys 20A to 20D, which stabilizes the controllability when the support 14 is turned. More specifically, when the support 14 is bent from the state illustrated in FIG. 16 toward the second side S2, the wires 26 and 28 come in contact with the guide pulleys 20A and 20B, respectively. In addition, when the support 14 is bent from the state illustrated in FIG. 17 toward the first side S1, the wires 30 and 32 come in contact with the guide pulleys 20C and 20D, respectively. Thus, in either case, such a state in which none of the wires are in contact with the guide pulleys 20A to 20D (run over the guide pulleys 20A to 20D) is avoided. As a result, the controllability when the support 14 is bent about the first rotating shaft 16 is stabilized.

While the present invention has been described above with reference to the embodiment, the present invention is not limited to the embodiment, and any combination or substitution of components in the embodiment as appropriate is included in the present invention. In addition, modifications such as combinations, changes in the order of processes, and various changes in design in the embodiment may be made on the embodiment on the basis of knowledge of a person skilled in the art, and such modified embodiments may be within the scope of the present invention.

### Industrial Applicability

The present invention can be used for a manipulator of a surgical robot.

### Reference Signs List

- 10: forceps device

- 12a, 12b: grasping portion
- 14: support
- 16: first rotating shaft
- 18: base member
- 20, 20A, 20B, 20C, 20D: guide pulley
- 22: second rotating shaft
- 24, 24a, 24b, 24c, 24d: jaw pulley
- 26, 28, 30, 32: wire
- 34, 34a, 34b, 34c, 34d: guide pulley
- 38: wire
- 42: support pulley

## Claims

1. A forceps device comprising:
a grasping part;
a support that holds the grasping part;
a first rotating shaft that turnably supports the support;
a base member that holds the first rotating shaft;
a plurality of guide pulleys arranged coaxially with the first rotating shaft;
a plurality of grasping portion wires running over the guide pulleys and the grasping part without other pulleys therebetween and transmitting driving force to move the grasping part;
a support pulley provided on the support; and
a support wire running over the support pulley and transmitting driving force to turn the support about the first rotating shaft.

2. The forceps device according to claim 1, wherein
the grasping part includes a first grasping portion and a second grasping portion configured to move relative to each other to grasp an object,
the first grasping portion is continuous with a first grasping portion pulley and a second grasping portion pulley, the first grasping portion pulley and the second grasping portion pulley constituting a bifurcated shape,
the second grasping portion is continuous with a third grasping portion pulley and a fourth grasping portion pulley, the third grasping portion pulley and the fourth grasping portion pulley constituting a bifurcated shape, and
the forceps device further comprising a second rotating shaft that rotatably supports the first grasping portion pulley, the third grasping portion pulley, the second grasping portion pulley, and the fourth grasping portion pulley arranged in this order.

3. A forceps device comprising:
a grasping part;
a support that holds the grasping part;
a first rotating shaft that turnably supports the support;
a base member that holds the first rotating shaft;
a plurality of guide pulleys arranged coaxially with the first rotating shaft; and
a plurality of grasping portion wires running over the guide pulleys and the grasping part without other pulleys therebetween and transmitting driving force to move the grasping part, wherein
the grasping part includes a first grasping portion and second grasping portion that move relative to each other to grasp an object,
the first grasping portion is continuous with a first grasping portion pulley and a second grasping portion pulley, the first grasping portion pulley and the second grasping portion pulley constituting a bifurcated shape,
the second grasping portion is continuous with a third grasping portion pulley and a fourth grasping portion pulley, the third grasping portion pulley and the fourth grasping portion pulley constituting a bifurcated shape, and
the forceps device further comprising a second rotating shaft that rotatably supports the first grasping portion pulley, the third grasping portion pulley, the second grasping portion pulley, and the fourth grasping portion pulley arranged in this order.

4. The forceps device according to claim 2 or 3, wherein
the support includes a cylindrical part through which the first rotating shaft extends, and
the guide pulleys are rotatably supported by an outer circumference of the cylindrical part.

5. The forceps device according to claim 4, wherein
the guide pulleys include first to fourth guide pulleys, and
the grasping portion wires are passed over the first to fourth guide pulleys and the first to fourth grasping portion pulleys without other pulleys therebetween.

6. The forceps device according to claim 5, wherein
the grasping portion wires include:
a first grasping portion wire running over the first guide pulley and the third grasping portion pulley;
a second grasping portion wire running over the second guide pulley and the first grasping portion pulley;
a third grasping portion wire running over the third guide pulley and the second grasping portion pulley; and
a fourth grasping portion wire running over the fourth guide pulley and the fourth grasping portion pulley.

7. The forceps device according to claim 6, wherein
the first grasping portion wire is passed over a first side of the first guide pulley with respect to a vertical cross section including the first rotating shaft and being perpendicular to the second rotating shaft, and then fixed to the third grasping portion pulley located on the first side with respect to the vertical cross section,
the second grasping portion wire is passed over the first side of the second guide pulley with respect to the vertical cross section, and then fixed to the first grasping portion pulley located on the first side with respect to the vertical cross section,
the third grasping portion wire is passed over a second side of the third guide pulley with respect to the vertical cross section, and then fixed to the second grasping portion pulley located on the second side with respect to the vertical cross section, and
the fourth grasping portion wire is passed over the second side of the fourth guide pulley with respect to the vertical cross section, and then fixed to the fourth grasping portion pulley with respect to the vertical cross section.

8. The forceps device according to claim 7, further comprising:
a fifth guide pulley located on an upstream side of the first guide pulley and on the second side with respect to the vertical cross section;
a sixth guide pulley located on an upstream side of the second guide pulley and on the second side with respect to the vertical cross section;
a seventh guide pulley located on an upstream side of the third guide pulley and on the first side with respect to the vertical cross section; and
an eighth guide pulley located on an upstream side of the fourth guide pulley and on the first side with respect to the vertical cross section.
